(19) **Europäisches Patentamt / European Patent Office / Office européen des brevets**

(11) **EP 4 678 229 A1**

(12) **EUROPEAN PATENT APPLICATION**

(43) Date of publication:
**14.01.2026 Bulletin 2026/03**

(21) Application number: **25188136.3**

(22) Date of filing: **08.07.2025**

(51) International Patent Classification (IPC):
**A61N 5/10** *(2006.01)*

(52) Cooperative Patent Classification (CPC):
**A61N 5/1031; A61N 5/1047**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **11.07.2024 US 202418770078**

(71) Applicant: **Siemens Healthineers International AG**
**6312 Steinhausen (CH)**

(72) Inventors:
- **VALENZUELA, Daniel**
  **0440 Helsinki (FI)**
- **PELTOLA, Jarkko**
  **004300 Tuusula (FI)**

(74) Representative: **Mathisen & Macara LLP**
**Charta House**
**30-38 Church Street**
**Staines-upon-Thames TW18 4EP (GB)**

(54) **RADIATION TREATMENT PLAN OPTIMIZATION METHOD AND APPARATUS**

(57)     A control circuit 101 accesses 201 at least one optimization factor and optimizes 203 a radiation treatment plan by, at least in part, automatically selecting at least one treatment field angle as a function of that at least one optimization factor as well as a redundancy cost constraint.

FIG. 2

EP 4 678 229 A1

**Description**

TECHNICAL FIELD

These teachings relate generally to treating a patient's planning target volume with energy pursuant to an energy-based treatment plan and more particularly to optimizing an energy-based treatment plan.

BACKGROUND

The use of energy to treat medical conditions comprises a known area of prior art endeavor. For example, radiation therapy comprises an important component of many treatment plans for reducing or eliminating unwanted tumors. Unfortunately, applied energy does not inherently discriminate between unwanted material and adjacent tissues, organs, or the like that are desired or even critical to continued survival of the patient. As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform parameters during each of a plurality of sequential fields. Treatment plans for radiation treatment sessions are often automatically generated through a so-called optimization process. As used herein, "optimization" will be understood to refer to improving a candidate treatment plan without necessarily ensuring that the optimized result is, in fact, the singular best solution. Such optimization often includes automatically adjusting one or more physical treatment parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

Treatment field angles often comprise one such treatment-platform parameter. While the number of fields may or may not be fixed, the irradiation angle may be varied from one field to the next. That irradiation angle is often determined by a gantry angle, a patient-support platform angle, or both. In many cases, the patient-support platform remains stationary, with the gantry angle being the only angle that changes during the treatment plan. Accordingly, a typical treatment field angle in practice is a two-dimensional vector having the gantry and patient-support platform angle coordinates as components (where, in the most general case of keeping the patient-support platform stationary (and hence 0 degrees), only the gantry angle contributes to a changing metric.)

SUMMRAY

In one aspect, the present invention provides a method as defined in claim 1. Optional features are specified in the dependent claims.

In one aspect, the present invention provides an apparatus as defined in claim 9. Optional features are specified in the dependent claims.

BRIEF DESCRIPTION OF DRAWINGS

The corresponding needs of the aforementioned application setting are at least partially met through provision of the radiation treatment plan optimization method and apparatus described in the following detailed description, particularly when studied in conjunction with the drawings, wherein:

FIG. 1 comprises a block diagram as configured in accordance with various embodiments of these teachings;
FIG. 2 comprises a flow diagram as configured in accordance with various embodiments of these teachings;
FIG. 3 comprises a schematic view as configured in accordance with various embodiments of these teachings;
FIG. 4 comprises a schematic view as configured in accordance with various embodiments of these teachings;
FIG. 5 comprises a schematic view as configured in accordance with various embodiments of these teachings;
FIG. 6 comprises a schematic view as configured in accordance with various embodiments of these teachings;
FIG. 7 comprises a schematic view as configured in accordance with various embodiments of the invention;
FIG. 8 comprises a schematic view as configured in accordance with various embodiments of the invention;
FIG. 9 comprises a schematic view as configured in accordance with various embodiments of the invention; and
FIG. 10 comprises a schematic view as configured in accordance with various embodiments of the invention.

Elements in the figures are illustrated for simplicity and clarity and have not necessarily been drawn to scale. For example, the dimensions and/or relative positioning of some of the elements in the figures may be exaggerated relative to other elements to help to improve understanding of various embodiments of the present teachings. Also, common but well-understood elements that are useful or necessary in a commercially feasible embodiment are often not depicted in order to facilitate a less obstructed view of these various embodiments of the present teachings. Certain actions and/or steps may be described or depicted in a particular order of occurrence while those skilled in the art will understand that such specificity with respect to sequence is not actually required. The terms and expressions used herein have the ordinary technical meaning as is accorded to such terms and expressions by persons skilled in the technical field as set forth above except where different specific meanings have otherwise been set forth herein. The word "or" when used herein shall be interpreted as having a disjunctive construction rather than a conjunctive construction unless otherwise specifically indicated.

DETAILED DESCRIPTION

Generally speaking, pursuant to these various embodiments, a control circuit accesses at least one optimization factor and optimizes a radiation treatment plan by, at least in part, automatically selecting at least one treatment field angle as a function of that at least one optimization factor as well as a redundancy cost constraint.

These teachings will accommodate a variety of approaches as regards the aforementioned optimization factor. By one approach, the at least one optimization factor corresponds to a patient's anatomical geometry. For example, the at least one optimization factor that corresponds to a patient's anatomical geometry may comprise at least one metric representing, on a field-by-field basis and from a beam's eye point of view, overlap between a patient's target volume and at least one protected volume.

In lieu of the foregoing or in combination therewith, the at least one optimization factor that corresponds to a patient's anatomical geometry may comprise at least one metric representing, on a field-by-field basis and from a beam's eye point of view, a quantity of voxels that are exposed to radiation. By one approach, the aforementioned quantity of voxels may comprise an average number of voxels per beamlet. By another approach, in lieu of the foregoing or in combination therewith, the quantity of voxels that are exposed to radiation may comprise a quantity of voxels that are exposed to radiation by a single beamlet from amongst a plurality of beamlets that comprise a therapeutic radiation beam.

By yet another approach, the at least one optimization factor that corresponds to a patient's anatomical geometry may comprise at least one metric representing, on a field-by-field basis and from a beam's eye point of view, both overlap between a patient's target volume and at least one protected volume as well as a quantity of voxels that are exposed to radiation.

The aforementioned redundancy cost constraint serves, at least in part, to help avoid the redundancy in optimal angles that would otherwise typically result due to the aforementioned optimization factor. The redundancy cost constraint will not ordinarily serve to reduce the total number of fields, but instead will urge the optimal field angles apart from one another to thereby achieve an increased diversity amongst those angles than would otherwise likely result.

Accordingly, this redundancy cost constraint will typically be a function of the field angles in some form. In particular, the redundancy cost constraint represents an optimization cost that is imposed on redundant selections of treatment field angles, such that optimization of the radiation treatment plan will tend to favor fewer of any particular treatment field angle as versus more of any given treatment field angle.

These teachings are highly flexible in practice and will accommodate any of a variety of modifications and/or supplemental features. By one approach, for example, one or both of the optimization factor(s) and the redundancy cost constraint can be weighted.

So configured, these teachings will support considerable (or total) automation in these regards, and will favorably leverage available information regarding the patient's geometry. These teachings can avoid the use of templates and are often able to deliver a viable solution within only a few seconds.

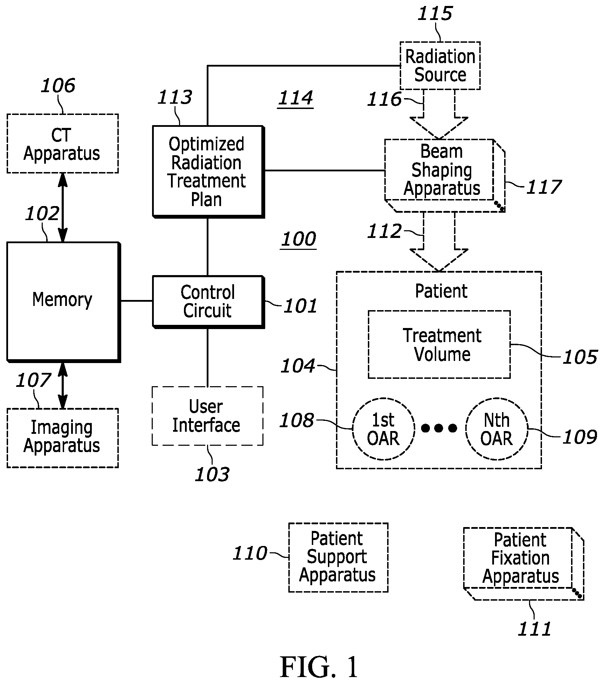

These and other benefits may become clearer upon making a thorough review and study of the following detailed description. Referring now to the drawings, and in particular to FIG. 1, an illustrative apparatus 100 that is compatible with many of these teachings will first be presented.

In this particular example, the enabling apparatus 100 includes a control circuit 101. Being a "circuit," the control circuit 101 therefore comprises structure that includes at least one (and typically many) electrically-conductive paths (such as paths comprised of a conductive metal such as copper or silver) that convey electricity in an ordered manner, which path(s) will also typically include corresponding electrical components (both passive (such as resistors and capacitors) and active (such as any of a variety of semiconductor-based devices) as appropriate) to permit the circuit to effect the control aspect of these teachings.

Such a control circuit 101 can comprise a fixed-purpose hard-wired hardware platform (including but not limited to an application-specific integrated circuit (ASIC) (which is an integrated circuit that is customized by design for a particular use, rather than intended for general-purpose use), a field-programmable gate array (FPGA), and the like) or can comprise a partially or wholly-programmable hardware platform (including but not limited to microcontrollers, microprocessors, and the like). These architectural options for such structures are well known and understood in the art and require no further description here. This control circuit 101 is configured (for example, by using corresponding programming as will be well understood by those skilled in the art) to carry out one or more of the steps, actions, and/or functions described herein.

It will be appreciated that the control circuit 101 may comprise a single integrated platform or may comprise a plurality of such circuits that work in cooperation with one another.

The control circuit 101 operably couples to a memory 102. This memory 102 may be integral to the control circuit 101 or can be physically discrete (in whole or in part) from the control circuit 101 as desired. This memory 102 can also be local with respect to the control circuit 101 (where, for example, both share a common circuit board, chassis, power supply, and/or housing) or can be partially or wholly remote with respect to the control circuit 101 (where, for example, the memory 102 is physically located in another facility, metropolitan area, or even country as compared to the control circuit 101). As with the control circuit 101, the memory 102 may comprise a singular structure or may comprise a plurality of memory platforms that collectively comprise the "memory" of this apparatus 100.

In addition to information such as optimization information for a particular patient, information regarding a particular radiation treatment platform as described herein, one or more optimization factors, and a redundancy cost constraint, this memory 102 can serve, for example, to non-transitorily store the computer instructions that, when executed by the control circuit 101, cause the control circuit 101 to behave as described herein. (As used herein, this reference to "non-transitorily" will be understood to refer to a non-ephemeral state for the stored contents (and hence excludes when the stored contents merely constitute signals or waves) rather than volatility of the storage media itself and hence includes both non-volatile memory (such as read-only memory (ROM) as well as volatile memory (such as a dynamic random access memory (DRAM).)

By one optional approach the control circuit 101 also operably couples to a user interface 103. This user interface 103 can comprise any of a variety of user-input mechanisms (such as, but not limited to, keyboards and keypads, cursor-control devices, touch-sensitive displays, speech-recognition interfaces, gesture-recognition interfaces, and so forth) and/or user-output mechanisms (such as, but not limited to, visual displays, audio transducers, printers, and so forth) to facilitate receiving information and/or instructions from a user and/or providing information to a user.

If desired the control circuit 101 can also operably couple to a network interface (not shown). So configured the control circuit 101 can communicate with other elements (both within the apparatus 100 and external thereto) via the network interface. Network interfaces, including both wireless and non-wireless platforms, are well understood in the art and require no particular elaboration here.

By one approach, a computed tomography apparatus 106 and/or other imaging apparatus 107 as are known in the art can source some or all of any desired patient-related imaging information.

In this illustrative example the control circuit 101 is configured to ultimately output an optimized energy-based treatment plan (such as, for example, an optimized radiation treatment plan 113). This energy-based treatment plan typically comprises specified values for each of a variety of treatment-platform parameters during each of a plurality of sequential exposure fields. In this case the energy-based treatment plan is generated through an optimization process, examples of which are provided further herein.

By one approach the control circuit 101 can operably couple to an energy-based treatment platform 114 that is configured to deliver therapeutic energy 112 to a corresponding patient 104 having at least one treatment volume 105 and also one or more organs-at-risk (represented in FIG. 1 by a first through an Nth organ-at-risk 108 and 109) in accordance with the optimized energy-based treatment plan 113. These teachings are generally applicable for use with any of a wide variety of energy-based treatment platforms/apparatuses. In a typical application setting the energy-based treatment platform 114 will include an energy source such as a radiation source 115 of ionizing radiation 116.

By one approach this radiation source 115 can be selectively moved via a gantry along an arcuate pathway (where the pathway encompasses, at least to some extent, the patient themselves during administration of the treatment). The arcuate pathway may comprise a complete or nearly complete circle as desired. By one approach the control circuit 101 controls the movement of the radiation source 115 along that arcuate pathway, and may accordingly control when the radiation source 115 starts moving, stops moving, accelerates, de-accelerates, and/or a velocity at which the radiation source 115 travels along the arcuate pathway.

As one illustrative example, the radiation source 115 can comprise, for example, a radio-frequency (RF) linear particle accelerator-based (linac-based) x-ray source. A linac is a type of particle accelerator that greatly increases the kinetic energy of charged subatomic particles or ions by subjecting the charged particles to a series of oscillating electric potentials along a linear beamline, which can be used to generate ionizing radiation (e.g., X-rays) 116 and high energy electrons.

A typical energy-based treatment platform 114 may also include one or more support apparatuses 110 (such as a couch) to support the patient 104 during the treatment session, one or more patient fixation apparatuses 111, a gantry or other movable mechanism to permit selective movement of the radiation source 115, and one or more energy-shaping apparatuses (for example, beam-shaping apparatuses 117 such as jaws, multi-leaf collimators, and so forth) to provide selective energy shaping and/or energy modulation as desired.

In a typical application setting, it is presumed herein that the patient support apparatus 110 is selectively controllable to move in any direction (i.e., any X, Y, or Z direction) during an energy-based treatment session by the control circuit 101. As the foregoing elements and systems are well understood in the art, further elaboration in these regards is not provided here except where otherwise relevant to the description.

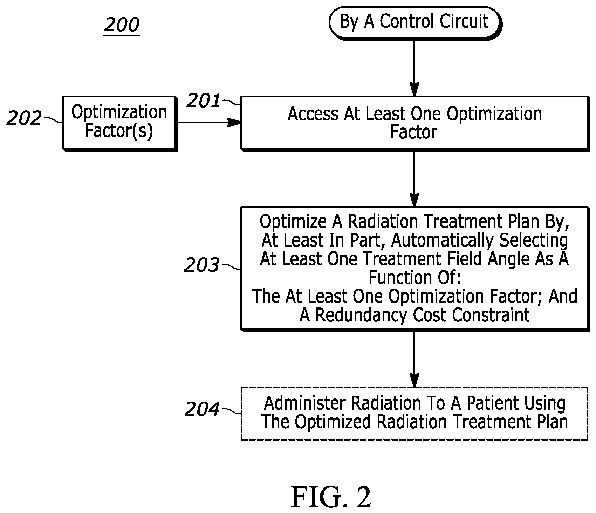

Referring now to FIG. 2, a process 200 that can be carried out, for example, in conjunction with the above-described application setting (and more particularly via the aforementioned control circuit 101) will be described. Generally speaking, this process 200 serves to facilitate generating an optimized radiation treatment plan 113 to thereby facilitate treating a particular patient with therapeutic radiation using a particular radiation treatment platform per that optimized radiation treatment plan.

At block 201, this process 200 provides for the control circuit 101 accessing (by accessing, for example, the aforementioned memory 102) at least one optimization factor 202. By one approach, the at least one optimization factor 202 corresponds to a patient's anatomical geometry. That anatomical geometry can be derived, for example, by accessing patient image information (as obtained, for example, via the aforementioned CT apparatus 106 and/or the aforementioned imaging apparatus 107). That anatomical geometry may represent, for example, relative sizes and positions of a target volume and a protected volume (or volumes) from various fields of view and hence can indicate when these two volumes overlap with one another (and to what extent) in a particular field of view.

Accordingly, the at least one optimization factor 202 may comprise at least one metric representing, on a field-by-field basis and from a beam's eye point of view, overlap between a patient's target volume 105 and at least one protected volume (such as a so-called organ-at-risk).

In lieu of the foregoing or in combination therewith, the at least one optimization factor 202 may comprise at least one metric representing, on a field-by-field basis and from a beam's eye point of view, a quantity of voxels that will be exposed to radiation. A voxel comprises the smallest distinguishable three-dimensional unit of space within a patient's body that is used for analytical purposes. (A voxel is sometimes thought of as the three-dimensional equivalent of a pixel in a two-dimensional image.) When creating a detailed map of a patient's anatomy for precise delivery of radiation therapy, voxels are often considered because they allow for an accurate calculation of the dose distribution within the body.

By one approach, the aforementioned quantity of voxels may comprise an average number of voxels per beamlet. A beamlet will be understood to refer to a small, individual ray or stream of radiation that is part of a larger configuration of radiation beams used in radiotherapy. The beamlet concept is akin to a pixel in a digital image, representing a minute portion of the overall treatment field. By adjusting the intensity and angle of each beamlet, clinicians can meticulously shape the radiation dose to conform to the three-dimensional geometry of the target tumor while minimizing exposure to surrounding healthy tissue.

By another approach, in lieu of the foregoing or in combination therewith, the quantity of voxels that are exposed to radiation may comprise a quantity of voxels that are exposed to radiation by a single beamlet from amongst a plurality of beamlets that together collectively comprise a therapeutic radiation beam.

By yet another approach, the at least one optimization factor that corresponds to a patient's anatomical geometry may comprise at least one metric representing, on a field-by-field basis and from a beam's eye point of view, both overlap between a patient's target volume and at least one protected volume as well as a quantity of voxels that are exposed to radiation.

At block 201, the control circuit 101 optimizes a radiation treatment plan by, at least in part, automatically selecting at least one treatment field angle as a function of the foregoing at least one optimization factor 202 as well as a redundancy cost constraint. "Cost constraints," in the context of iterative optimization approaches, In the context of an iterative optimization method, refer to a predefined limitation that regulates the extent of one or more resources that can be allocated towards achieving the desired optimization objectives. Such constraints are factored into the optimization process to ensure that the solution adheres to boundaries set by such constraints. Accordingly, while the optimization process iteratively searches for an optimal solution by adjusting variables and assessing outcomes, the process simultaneously ensures that the total cost incurred in reaching that solution does not exceed the set cost constraint, thereby balancing the twin goals of optimization and management of the concern represented by the cost constraint.

The aforementioned redundancy cost constraint will typically be a function of the field angles in some selected form. In particular, the redundancy cost constraint represents an optimization cost that is imposed on redundant selections of any given treatment field angles, such that optimization of the radiation treatment plan will tend to favor fewer of any one particular treatment field angle while tending to favor more of a variety of different treatment field angles.

By one approach, one or both of the optimization factor(s) 202 and the redundancy cost constraint can be weighted. Such weighting will be understood to involve assigning varying levels of importance to different components of the problem being solved. By adjusting these weights, the optimization process can prioritize certain factors over others, effectively shaping the solution space and guiding the optimization process toward the most desirable outcome.

At optional block 204, the resultant optimized radiation treatment plan 113 can be used to administer radiation 112 to a patient 104 via a corresponding radiation treatment platform 114 as described above

Further details that comport with these teachings will now be presented. It will be understood that the specific details of these examples are intended to serve an illustrative purpose and are not intended to suggest any particular limitations with respect to these teachings.

Generally speaking, these teachings can provide for automatically selecting a subset of directions that are preferable to employ while applying radiation. This could correspond, for instance, to a set of directions for intensity-modulated radiation therapy beams. By one approach, a general formulation can be presented as follows for a set of entry beams $B_1, B_2, ..., B_n$ (where "n" is an integer) that are situated either in a coplanar arc or from the whole three-dimensional space. In this case, one can define the following cost:

$$C(B_1, B_2, ..., B_n) = \sum_1^n G(B_i) + \sum_1^n R(B_i)$$

In the foregoing equation, $G(B_i)$ represents the geometrical cost of irradiating from the direction of $B_i$, based on the patient anatomy. This metric can model different benefits and drawbacks of choosing one direction as versus a different direction.

As one illustrative example, one may consider two criteria that are modelled by two different functions. The first function could be the amount of overlap between the targets and the organs at risks when projected in the direction defined by $B_i$. The applicant has determined that the directions where there is less overlap between the target and organs-at-risk can generally be more beneficial (all other things being equal), and therefore these should be preferred. This overlapping can be represented by $O(B_i)$.

These teachings will accommodate defining this overlap function in a variety of ways. One approach can be:

$$O(B_i) = \sum_{pixel\ in\ 2D\ proyection} f(\{p \mid p \in Target \wedge p \in Organ\})$$

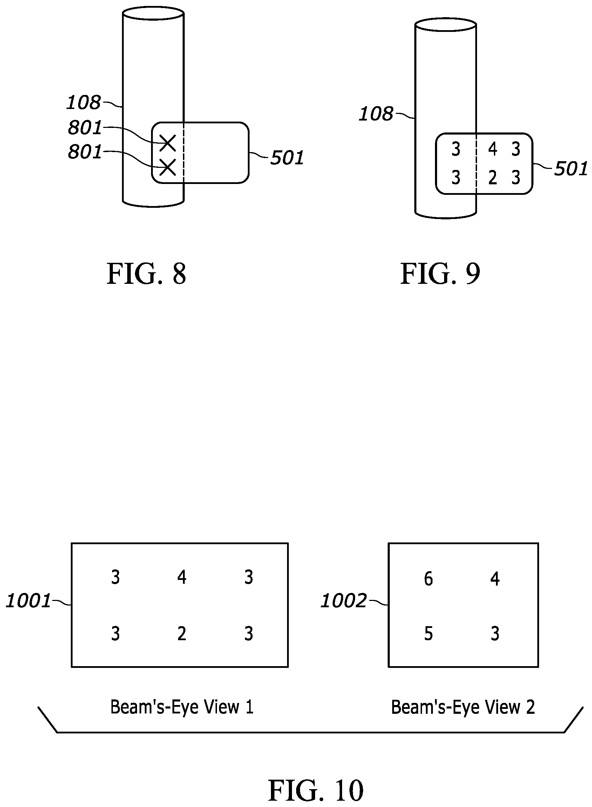

Referring to FIG. 8, one simple example of this function f would be the size of the set that is the total amount of voxels 801 on the beam's-eye view two-dimensional projection that simultaneously belong to both the target volume 105 and the corresponding organ-at-risk 108.

Referring to FIG. 9, by another approach the function f would be the sum of the projected organ volume into each voxel that simultaneously belongs to both the target volume 105 and the corresponding organ-at-risk 108. In this illustrative example, that sum would be "6" (i.e., the resultant sum of "3" and "3").

These teachings will readily accommodate different target volumes and different organs-at-risk having different volumes as well as differing levels of importance in the plan objectives. For example, by one approach, these teachings will accommodate including normalization factors. The following equation represents one example in such regards.

$$O(B_i) = \sum_{T_x \in Targets} \lambda \sum_{Org_y \in Organs} \mu \sum_{pixel\ p\ in\ 2D\ proyection} f(\{p \mid p \in T_x \wedge p \in Org_y\})$$

The second function could be the average amount of voxels that can be irradiated from a single beamlet. That is, this metric would favor choosing angles from which it is possible to irradiate most of the target using the least number of beamlets. This function can be denoted as $V(B_i)$.

Conceptually, this function attempts to capture the longitudinal efficiency of the angle. This approach can be formalized as follows:

$$V(B_i) = \sum_{pixel\ in\ 2D\ proyection} g(\{p \mid p \in Target\})$$

Referring to FIG. 10, as one illustrative example in these regards, this function can be the average values of the target projection on the beam's-eye view two-dimensional projection. FIG. 10 presents a first beam's eye view projection 1001 and a second, different beam's eye view projection 1002. In this example, the average is higher on the second beam's-eye view projection 1002, so that would be the preferred direction.

Accordingly,

$$G(B_i) = O(B_i) + V(B_i).$$

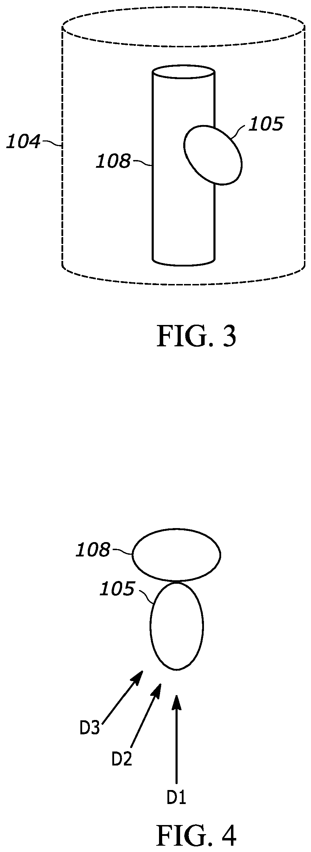

FIG. 3 presents a schematic representation of a patient 104 having both a target volume 105 (in this example, a tumor) and a volume to be protected 108. In this example, the target volume 105 has an organ-at-risk 108 nearby that follows a more elongated direction of the target volume 105. In this example, the process 200 considers three different radiation beamlet directions (D1, D2, and D3).

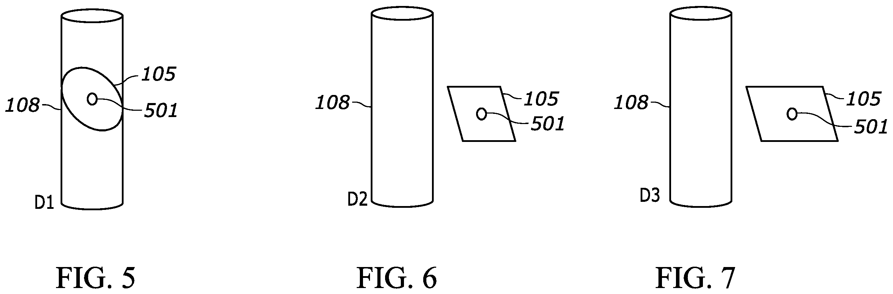

FIGS. 5, 6, and 7 show the beam's eye view (BEV) from directions D1, D2, and D3 respectively (with reference numeral 501 denoting a beamlet's center). It can be seen that if one only considers the overlap metric $O(B_i)$, D1 has the highest cost due to the high degree of overlap between the organ-at-risk 108 and the target volume 105, whereas both D2 and D3 have zero cost in these regards because there is no overlap between these volumes whatsoever. Therefore, if only the overlap metric were used, directions D2 and D3 would be considered equivalently good.

On the other hand, from the perspective of the voxel's metric $V(B_i)$, the best direction would be D1, the second best would be D2, and the worst direction would be D3. This result occurs because the increasing two-dimensional size of the target projection into the beam's-eye view from D1 to D3 implies a reduced number of average voxels that are radiated per beamlet.

A combination of both metrics, however, can favor direction D2 as a compromise between these two considerations.

These teachings are also able to take redundancy into account. If one only considers the anatomical geometry per the foregoing approach, the likely result may be that all of the chosen directions would be the same (or very nearly the same if one excludes repetitions). The present teachings will accommodate introducing a redundancy cost, $R(B_i)$. This cost measures the redundancy between the direction of the beam $B_i$ with respect to the other beams $B_j, \forall j! = i$ to thereby encourage irradiating the target volume from as different directions as possible.

As one illustrative example in these regards, let $\alpha_{ij} \in [0,\pi]$ be the angle between $B_i$ and $B_j$. One can then define $R(B_i)$ as the dot product between the vectors that correspond to the entry directions:

$$R(B_i) = \sum_{j \,!=i}^{n} B_i \cdot B_j = \left\| \sum_{j\,!=i}^{n} \cos(\alpha_{ij}) \right\|$$

While a cosine function naturally provides a measure of orthogonality/parallelism between different entry angles, other functional forms can be used as well. For example, linear or gaussians functions may be beneficially applied.

As noted above, weighting can be employed if desired. As one example in these regards, one can use specific weights for each of the components, resulting in the following formulation:

$$C(B_1, B_2, ..., B_n) = \alpha \sum_{1}^{n} O(B_i) + \beta \sum_{1}^{n} V(B_i) + \gamma \sum_{1}^{n} R(B_i)$$

By one approach, the specific values for weights of $\alpha$, $\beta$, and $\gamma$ can be left as user-defined parameters. Alternatively, these teachings will accommodate determining their values by taking a series of different expert-set up entry angles and choosing the weights that can minimize the prediction error between the model and the expert-defined angles.

These teachings will accommodate considering the number of desired directions n as an input parameter. One can also choose an initially-suitable resolution to sample the angle space (for example, 5 degrees, 10 degrees, 15 degrees, or the like).

These teachings will also accommodate precomputing the anatomical values for each direction $O(B_i) + V(B_i)$. It can then be computationally fast to simply compute the cost of all the combinations of n angles over the sampled angle space. That will provide an initial candidate set. If desired, one can then do a second round, to accommodate achieving as fine a resolution as might be desired. This approach will permit, for example, exploring many or all of the combinations where each of the initial solutions can be modified within the resolution of the first iteration.

In many typical prior art approaches, the user sets angle directions manually or, in the alternative, makes use of site-specific templates. Such approaches fail to offer the opportunity to achieve the benefits provided by the present teachings.

Further aspects of the invention are provided by the subject matter of the following clauses:

Clause 1. A method comprising: by a control circuit: accessing at least one optimization factor; optimizing a radiation treatment plan by, at least in part, automatically selecting at least one treatment field angle as a function of: the at least one optimization factor; and a redundancy cost constraint.

Clause 2. Any combination of any clause presented herein wherein the at least one optimization factor corresponds to a patient's anatomical geometry.

Clause 3. Any combination of any clause presented herein wherein the at least one optimization factor that corresponds to a patient's anatomical geometry comprises at least one metric representing, on a field-by-field basis and from a beam's eye point of view, overlap between a patient's target volume and at least one protected volume.

Clause 4. Any combination of any clause presented herein wherein the at least one optimization factor that corresponds to a patient's anatomical geometry comprises at least one metric representing, on a field-by-field basis and from a beam's eye point of view, a quantity of voxels that are exposed to radiation.

Clause 5. Any combination of any clause presented herein wherein the quantity of voxels comprises an average number of voxels per beamlet.

Clause 6. Any combination of any clause presented herein wherein the quantity of voxels that are exposed to radiation comprises a quantity of voxels that are exposed to radiation by a single beamlet from amongst a plurality of beamlets that comprise a therapeutic radiation beam.

Clause 7. Any combination of any clause presented herein wherein the at least one optimization factor that corresponds to a patient's anatomical geometry comprises at least one metric representing, on a field-by-field basis and from a beam's eye point of view, both: overlap between a patient's target volume and at least one protected volume; and a quantity of voxels that are exposed to radiation.

Clause 8. Any combination of any clause presented herein wherein the redundancy cost constraint represents an optimization cost imposed on redundant selections of treatment field angles, such that optimization of the radiation treatment plan favors fewer of a particular treatment field angle as versus more of the treatment field angle.

Clause 9. Any combination of any clause presented herein wherein at least one of the at least one optimization factor and the redundancy cost constraint are weighted.

Clause 10. Any combination of any clause presented herein wherein both the at least one optimization factor and the redundancy cost constraint are weighted.

Clause 11. An apparatus comprising: a control circuit configured to: access at least one optimization factor; and optimize a radiation treatment plan by, at least in part, automatically selecting at least one treatment field angle as a function of: the at least one optimization factor; and a redundancy cost constraint.

Clause 12. Any combination of any clause presented herein wherein the at least one optimization factor corresponds to a patient's anatomical geometry.

Clause 13. Any combination of any clause presented herein wherein the at least one optimization factor that corresponds to a patient's anatomical geometry comprises at least one metric representing, on a field-by-field basis and from a beam's eye point of view, overlap between a patient's target volume and at least one protected volume.

Clause 14. Any combination of any clause presented herein wherein the at least one optimization factor that corresponds to a patient's anatomical geometry comprises at least one metric representing, on a field-by-field basis and from a beam's eye point of view, a quantity of voxels that are exposed to radiation.

Clause 15. Any combination of any clause presented herein wherein the quantity of voxels comprises an average number of voxels per beamlet.

Clause 16. Any combination of any clause presented herein wherein the quantity of voxels that are exposed to radiation comprises a quantity of voxels that are exposed to radiation by a single beamlet from amongst a plurality of beamlets that comprise a therapeutic radiation beam.

Clause 17. Any combination of any clause presented herein wherein the at least one optimization factor that corresponds to a patient's anatomical geometry comprises at least one metric representing, on a field-by-field basis and from a beam's eye point of view, both: overlap between a patient's target volume and at least one protected volume; and a quantity of voxels that are exposed to radiation.

Clause 18. Any combination of any clause presented herein wherein the redundancy cost constraint represents an optimization cost imposed on redundant selections of the treatment fields, such that optimization of the radiation treatment plan favors more treatment fields as versus fewer treatment fields.

Clause 19. Any combination of any clause presented herein wherein at least one of the at least one optimization factor and the redundancy cost constraint are weighted.

Clause 20. Any combination of any clause presented herein wherein both the at least one optimization factor and the redundancy cost constraint are weighted.

Those skilled in the art will recognize that a wide variety of modifications, alterations, and combinations can be made with respect to the above-described embodiments without departing from the scope of the invention, and that such modifications, alterations, and combinations are to be viewed as being within the ambit of the inventive concept.

**Claims**

1. A method comprising:

   by a control circuit:

   accessing at least one optimization factor;
   optimizing a radiation treatment plan by, at least in part, automatically selecting at least

   one treatment field angle as a function of:

   - the at least one optimization factor; and
   - a redundancy cost constraint.

2. The method of claim 1 wherein the at least one optimization factor corresponds to a patient's anatomical geometry.

3. The method of claim 2 wherein the at least one optimization factor that corresponds to a patient's anatomical geometry comprises at least one metric representing, on a field-by-field basis and from a beam's eye point of view, overlap between a patient's target volume and at least one protected volume.

4. The method of claim 2 wherein the at least one optimization factor that corresponds to a patient's anatomical geometry comprises at least one metric representing, on a field-by-field basis and from a beam's eye point of view, a quantity of voxels that are exposed to radiation.

5. The method of claim 4 wherein the quantity of voxels comprises an average number of voxels per beamlet.

6. The method of claim 4 wherein the quantity of voxels that are exposed to radiation comprises a quantity of voxels that are exposed to radiation by a single beamlet from amongst a plurality of beamlets that comprise a therapeutic radiation beam.

7. The method of claim 2 wherein the at least one optimization factor that corresponds to a patient's anatomical geometry comprises at least one metric representing, on a field-by-field basis and from a beam's eye point of view, both:

   overlap between a patient's target volume and at least one protected volume; and
   a quantity of voxels that are exposed to radiation.

8. The method of any one of claims 1 to 7 wherein the redundancy cost constraint represents an optimization cost imposed on redundant selections of treatment field angles, such that optimization of the radiation treatment plan favors fewer of a particular treatment field angle as versus more of treatment field angles that are different from the particular treatment field angle.

9. An apparatus comprising:
   a control circuit configured to:

   access at least one optimization factor; and
   optimize a radiation treatment plan by, at least in part, automatically selecting at least

   one treatment field angle as a function of:

   - the at least one optimization factor; and
   - a redundancy cost constraint.

10. The apparatus of claim 9 wherein the at least one optimization factor corresponds to a patient's anatomical geometry.

11. The apparatus of claim 10 wherein the at least one optimization factor that corresponds to a patient's anatomical geometry comprises at least one metric representing, on a field-by-field basis and from a beam's eye point of view:

    overlap between a patient's target volume and at least one protected volume; and/or

a quantity of voxels that are exposed to radiation.

12. The apparatus of claim 10 wherein the at least one optimization factor that corresponds to a patient's anatomical geometry comprises at least one metric representing, on a field-by-field basis and from a beam's eye point of view, a quantity of voxels that are exposed to radiation, and, optionally, wherein:

the quantity of voxels comprises an average number of voxels per beamlet, or
the quantity of voxels that are exposed to radiation comprises a quantity of voxels that are exposed to radiation by a single beamlet from amongst a plurality of beamlets that comprise a therapeutic radiation beam.

13. The apparatus of claim 9, 10, 11 or 12 wherein the redundancy cost constraint represents an optimization cost imposed on redundant selections of treatment field angles, such that optimization of the radiation treatment plan favors more differing treatment field angles as versus fewer similar treatment field angles.

14. The method or apparatus of any one of claims 1 to 13 wherein at least one of the at least one optimization factor and the redundancy cost constraint are weighted.

15. The method or apparatus of any one of claims 1 to 14 wherein both the at least one optimization factor and the redundancy cost constraint are weighted.

FIG. 1

_200_

By A Control Circuit

202～ Optimization Factor(s)   201～  →  Access At Least One Optimization Factor

203～ Optimize A Radiation Treatment Plan By, At Least In Part, Automatically Selecting At Least One Treatment Field Angle As A Function Of:
The At Least One Optimization Factor; And A Redundancy Cost Constraint

204～ Administer Radiation To A Patient Using The Optimized Radiation Treatment Plan

FIG. 2

104

105

108

## FIG. 3

108

105

D3

D2

D1

## FIG. 4

FIG. 5

FIG. 6

FIG. 7

EP 4 678 229 A1

FIG. 8

FIG. 9

Beam's-Eye View 1                    Beam's-Eye View 2

FIG. 10

Europäisches
Patentamt
European
Patent Office
Office européen
des brevets

# EUROPEAN SEARCH REPORT

**Application Number**

EP 25 18 8136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/051513 A1 (PUGACHEV ANDREI [US] ET AL) 2 May 2002 (2002-05-02) <br> * paragraphs [0017], [0056] * <br> * paragraphs [0065] - [0067] * <br> * paragraph [0073] * <br> ----- | 1-15 | INV. <br> A61N5/10 |
| X | US 2018/111005 A1 (RANGANATHAN VAITHEESWARAN [IN] ET AL) 26 April 2018 (2018-04-26) <br> * paragraphs [0003], [0015] * <br> * paragraphs [0037] - [0040] * <br> * paragraph [0085] * <br> ----- | 1,2,4, 9-12,14, 15 | |
| X | US 2018/154179 A1 (OLLILA SANTTU [FI] ET AL) 7 June 2018 (2018-06-07) <br><br> * paragraphs [0062] - [0071] * <br> ----- | 1-3, 8-11, 13-15 | |

**TECHNICAL FIELDS SEARCHED (IPC)**

A61N

The present search report has been drawn up for all claims

| Place of search | Date of completion of the search | Examiner |
|---|---|---|
| Munich | 22 October 2025 | Seiferle, Benedict |

EPO FORM 1503 03.82 (P04C01)

## ANNEX TO THE EUROPEAN SEARCH REPORT
## ON EUROPEAN PATENT APPLICATION NO.

EP 25 18 8136

This annex lists the patent family members relating to the patent documents cited in the above-mentioned European search report.
The members are as contained in the European Patent Office EDP file on
The European Patent Office is in no way liable for these particulars which are merely given for the purpose of information.

22-10-2025

| Patent document cited in search report | Publication date | Patent family member(s) | | Publication date |
|---|---|---|---|---|
| US 2002051513 A1 | 02-05-2002 | NONE | | |
| US 2018111005 A1 | 26-04-2018 | CN | 107666940 A | 06-02-2018 |
| | | EP | 3302699 A1 | 11-04-2018 |
| | | JP | 2018515274 A | 14-06-2018 |
| | | JP | 2021175513 A | 04-11-2021 |
| | | US | 2018111005 A1 | 26-04-2018 |
| | | WO | 2016188754 A1 | 01-12-2016 |
| US 2018154179 A1 | 07-06-2018 | EP | 3548143 A1 | 09-10-2019 |
| | | US | 2018154179 A1 | 07-06-2018 |
| | | WO | 2018104291 A1 | 14-06-2018 |

EPO FORM P0459

For more details about this annex : see Official Journal of the European Patent Office, No. 12/82